# EUROPEAN PATENT SPECIFICATION

(11) **EP 2 320 795 B1**
(45) Date of publication and mention of the grant of the patent: **23.07.2014**
(21) Application number: 09754305.2
(22) Date of filing: 29.05.2009
(51) Int. Cl.: A61B 5/103, A61B 5/00

(54) **DEVICE FOR MEASURING THE MECHANICAL PROPERTIES OF THE SKIN WITHOUT CONTACT WITH THE MEASUREMENT ZONE**
VORRICHTUNG ZUR MESSUNG DER MECHANISCHEN EIGENSCHAFTEN DER HAUT OHNE KONTAKT MIT DER MESSZONE
DISPOSITIF POUR MESURER LES PROPRIÉTÉS MÉCANIQUES DE LA PEAU SANS CONTACT AVEC LA ZONE DE MESURE

(30) Priority: 30.05.2008 FR 0853559
(43) Date of publication of application: 18.05.2011
(73) Proprietor: Sederma, 78610 Le Perray en Yvelines (FR)
(72) Inventor: Fournial, Arnaud, F-75016 Paris (FR); Mondon, Philippe, F-94230 Cachan (FR); Fache, Sébastien, F-75013 Paris (FR); Mas Chamberlin, Claire, F-78460 Chevreuse (FR)
(86) International application number: PCT/IB2009/052269
(87) International publication number: WO 2009/144680

(56) References cited:
- EP-A- 1 321 098
- WO-A-97/29686
- WO-A-2004/073514
- US-B1- 6 324 419

## Description

This invention relates to a device for measuring the mechanical properties of the skin. More particularly, the device according to the invention enables a high-frequency measurement of the mechanical properties of the skin that is free from all parameters interfering with measurement. Methods of measurement of the mechanical properties of the skin have been known for many years and their use, to evaluate, in particular, the state of healthy skin and the changes induced by cosmetic products have been widely reported in the scientific literature (Agache and Humbert, 2004).

The methods employ stretching, torsion, suction, compression or percussion implemented by various systems such as: the Cutometer (EP0362616 A1) and Ballistometer (FR 2 533 030 A1), etc.

However, in addition to the stress exerted on the skin, all the systems have the disadvantage of making direct contact with the measurement zone, thus giving rise to interfering parameters. The device in direct contact with the measurement zone exerts pressure on the skin that is liable to exert a tensile stress on elastic fibers or induce movements of fluid in the cutaneous tissue and thus change the mechanical parameters, resulting in a truncated measurement.

WO-A-2004/073514 discloses a device to assess vascular tissue damages. The device applies pressure on the vascular area to be analyzed so that the blood is evacuated from this zone for a short time. During this period, an optical system is used to evaluate the vascular damage. More specifically, the device comprises a compressed air nozzle used to inflate a membrane designed to apply pressure on the skin and through which the optical system performs the measurement. EP-A-1 321 098 discloses a device and a method for the evaluation of the eye contour, the device comprising means for studying the pigmentation of the region around the eye and means for studying the mechanical properties. A camera can be provided to light the measurement region for helping the acquisition of colorimetric data.

The means for evaluating the mechanical properties comprise a device for the mechanical evaluation and means for an acquisition of the relief.

According to one embodiment the device for the mechanical evaluation comprises a device for projecting compressed air and the device for the acquisition of the relief comprises a fringe acquisition system adapted to make a measurement every 3 or 4 seconds.

The technical problem to be solved by the inventors thus consisted in designing a measurement device able to evaluate the mechanical properties of the skin while remaining free from all the parameters interfering with measurement.

Thus the object of the present invention is a device for measuring the mechanical properties of the skin comprising:
a) a pneumatic assembly for delivering compressed air to apply a stress to the surface of the skin undergoing measurement,
b) an electronic assembly for contact-free measurements on said surface of the skin subjected to the stress, and
c) a control software for controlling the electronic assembly and for processing measurement data in order to deduce the mechanical properties of the skin.
   characterized in that the electronic assembly comprises a laser-diode laser-line profilometer comprising:
   - a laser-diode laser-line generator for projecting a laser line on said skin surface, and
   - a CMos or CCD image sensor for recording the evolution of the position of the points of said laser line points, said profilometer being adapted to measure the position of the laser line at frequencies of between 50 and 1000 Hz.

More particularly, the pneumatic assembly consists of an air compressor which supplies a nozzle via a pneumatically operated switch, a flow rate regulator and a pressure regulator.

According to a second aspect, the present invention is directed to a non-invasive and non-destructive method for measuring the mechanical surface and volume properties of the skin, characterised in that it comprises the following steps:
a) Applying a deforming stress to the skin surface under measurement by using an air jet with adjustable pressure and flow rate,
b) Projecting a laser line on the surface of the skin deformed by said stress,
c) Recording the position of the laser line points at a frequency of between 50 and 1000 Hz without contact with said skin surface,
d) Processing the data recorded in step c) using a software to deduce the mechanical, surface and volume properties of the skin.

The above features and advantages will be clearer in the following description and attached drawings, in which:

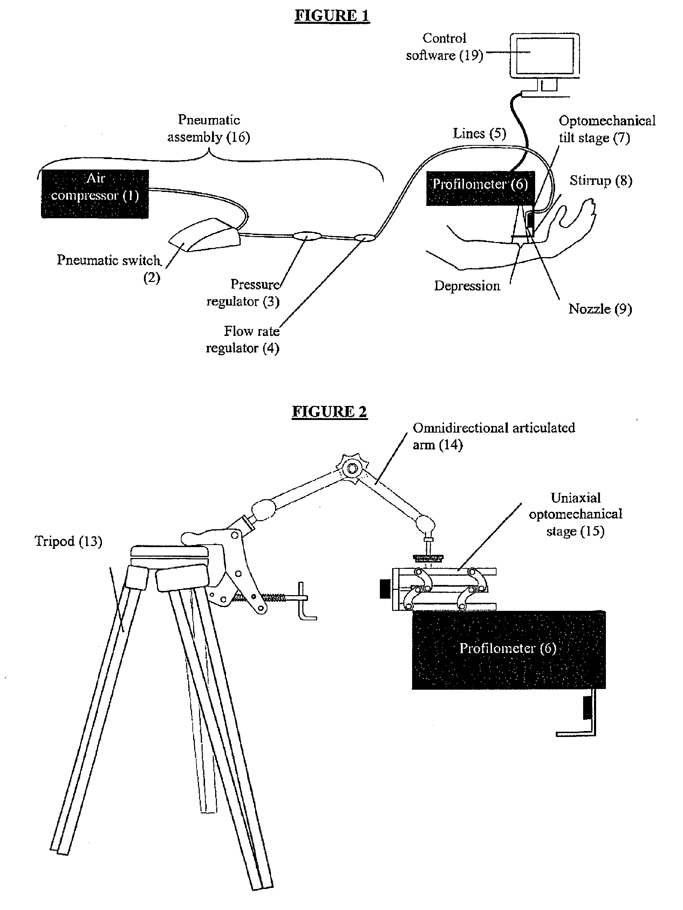

Figure 1 shows a side view of the device (the tripod, articulated arm and uniaxial optomechanical stage being not shown).
Figure 2 shows a side view of the profilometer fixation means.

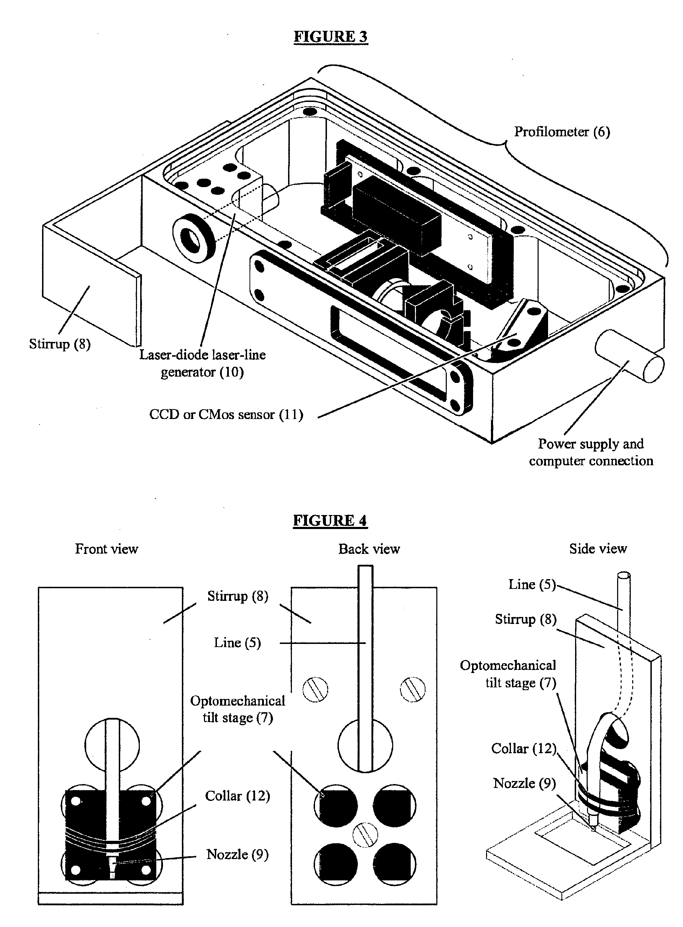

Figure 3 shows an interior side view of the profilometer.
Figure 4 shows a front, back and perspective side views of nozzle attachment to the stirrup.
Figure 5 shows the scheme of Burger's model.
Figure 6 shows the change in displacement of a system (such as the skin) whose behavior can be modeled using Burger's model.
Figure 7 shows the application of the compressed air stress to the skin as a function of time (t).
Figure 8 shows an example of a linear zone measured before applying the stress at a time t such that t₀ ≤t < t₁.
Figure 9 shows another example of the profile of the same linear zone during application of the stress at a time t such that t₁ < t < t₂.
Figure 10 shows an example of the profile of the linear area 10 ms after applying the stress, i.e. at t₃ = t₂ + 10 ms, the frequency being 100 Hz.
Figure 11 shows an example of the profile of said linear zone 5 s after applying the stress at t₄= t₂ + 5 s.
Figure 12 shows the change in the displacement profile of the linear zone, as a function of time, pre-, per- and post-compressed air stress.
Figure 13 shows the volume of cutaneous displacement.

More particularly, the device according to the present invention comprises in a laser-diode laser-line generator (10) and a CMos (complementary metal-oxide semiconductor) or CCD (charge-coupled device) (11) sensor that can be combined in a single apparatus in order to constitute a laser-line profilometer (6) able to measure at high frequencies. The expression 'high frequencies' refers to frequencies between 50 and 1000 Hz, preferably greater than or equal to 100 Hz, and, in particular, 250 Hz. In the context of the present invention, the expression 'high frequencies' indicates a measurement that is repeated in a time interval of between 20 ms and 1 ms, enabling access to continuous measurement.

The optical measurement is non-invasive, fast and very precise, the linearity of the profilometer being less than 15 microns (= µm) and, preferably, about 1 micron. The axial resolution of the profilometer is less than 5 microns and preferably about 1 micron.

The stress is induced on the measurement zone via compressed air delivered by an air compressor (1), pressure and flow rate being variable. In the context of the present invention, an 'air compressor' refers to an apparatus designed to increase the air or gas pressure in a container, this term also includes the use of pressurized cylinders.

Said air compressor (1) is connected by a semi-rigid line to a pneumatic switch (2) enabling control of stress application time as well as the profile on the software graphic interface. The switch may consist in a pedal, manual device, or solenoid valve that parameterizes the duration of the stress. According to the invention, the term 'stress' refers to the force exerted on the measurement zone per unit area.

In addition, to achieve the present invention, it is important that the flow rate and pressure of the air delivered by the compressor remain stable during measurement. Accordingly, said pneumatic switch is connected to a pressure regulator (3) which is in turn connected to a flow rate regulator (4). To overcome the problem of barometric variation, the pressure regulator (3) includes at least one micrometer screw enabling constant pressure to be maintained. The flow rate regulator (4) also includes at least one micrometer screw. The use of a regulator with micrometer screw is necessary for fine adjustment of the air flow.

The flow rate regulator (4) is then connected to a nozzle (9).

In order for the compressed air delivered by the nozzle to deform the skin at laser line level, an optomechanical tilt stage (7) is used. The optomechanical tilt stage (7) enables fine adjustment of the nozzle angle using a micrometer screw and hence orientation of the compressed air jet on the measurement line. Said nozzle is attached to the stage (7) using a collar (12). The stage is in turn attached to a stirrup (8) by one or several screws. The stirrup is screwed to the profilometer (6). Thus, according to another aspect of the invention, the nozzle is orientable via an optomechanical tilt stage (7).

The various components of the device are connected by semi-rigid lines (5).

Obviously, the order described is in no way limitative and the various components may be interchanged to ensure correct operation of the system.

The distance between the air nozzle and surface measured is between 1 mm and 10 cm or more and preferably about 1 cm. The form of the nozzle may be varied in order to modify the jet impacting the skin. A single nozzle or multiple nozzles may be used.

The present device also includes a uniaxial optomechanical translation stage (15) which is attached both directly to the profilometer (6) and to an omnidirectional articulated arm (14). The expression 'omnidirectional articulated arm' is taken to mean a system that enables the profilometer to be oriented in all directions.

Said articulated arm is then fixed to a tripod (13). Obviously, the tripod may be replaced by any other component ensuring satisfactory stability.

The articulated arm positions and fixes the device, then the uniaxial translation stage (15) enables the profilometer to be raised or lowered by vertical movement. The system is positioned above the measurement site so as to maintain a constant distance between the profilometer and the surface under study.

One of the advantages of the invention is thus the measurement without any contact between the device and the zone to which stress is applied. Therefore, the term 'contact-free' indicates that the device and components enable measurement and data collection without entering into contact with the zone under evaluation.

The profilometer is also connected to a computer running the software controlling the sensor assembly and recording the data.

The data are obtained by triangulation of a laser line in a system enabling modulation of measurement frequency. The lateral resolution is less than 2 mm and preferably between 10 and 100 µm. The data are transmitted to the computer via the data-acquisition software, and after processed using mathematical algorithms.

The software of the device thus enables high-frequency acquisition of cutaneous profiles and, more particularly, of a linear zone as a function of time.

Thus, the control software measures skin displacement as a function of time pre-, per- and post-stress application.

According to a preferred embodiment, the present device has the advantage of being small, light and thus easily transportable.

The device according to the present invention has a great flexibility; Thanks to the omnidirectional articulated arm (14), all the areas of the body may be evaluated: forehead, face, neck, forearm, hand, bust, abdomen, leg, foot, etc.

In another embodiment, the stress may be induced using any gas from a compressor or pressurized cylinder fitted with a pressure-reducing valve.

Measurement may also be implemented by any system able to measure a profile or full-field measurement, in a static and dynamic manner, on any surface.

More particularly, the present invention may also include an apparatus simultaneously measuring a number of defined points in a measurement zone using chromatic confocal imaging photometry and exploiting the axial chromatism of the lens system.

Accordingly, the present invention may incorporate an apparatus based on that technology such as:
- a point sensor with a wide measurement field range from 20 µm to 24 mm. Such sensor enables high axial precision, generally less than a micrometer. For example, the axial precision of an optical wand with a measurement field of one centimeter is 600 nanometers. The measurement frequently may reach 40 kHz;
- a line sensor reporting a surface profile in a single measurement. Such device enables contact-free surface topography and 3D-measurement. The measurement line is subdivided into 180 points and the measurement frequency is up to 1.8 kHz;
- chromatic confocal field sensor obtained by adding a scanning mirror in the line sensor optical head. Two models are available:
   - measurement field: 1.8 x 1.8 mm, 500 µm of measurement field, axial resolution of 125 nm and axial precision of 500 nm
   - measurement field: 44.75 x 44.75mm, 2 mm of measurement field, 500 nm of axial resolution and 2.5 µm of axial precision.

These two sensors enable one measurement every 130 ms.

In another aspect of the invention, the currently known devices in the cosmetic field and, more particularly, the devices used to measure the mechanical properties of the skin do not enable two-or three-dimensional measurements of a surface subjected to stress at high frequencies.

The devices, which are familiar to professionals specialized in cosmetic devices measuring the properties of the skin, are able to make high-frequency measurements only for a point located in the measurement zone. Examples include the Cutometer. There are also devices enabling measurement in two (= linear zone) or three dimensions but for a given time, for example the fringe-projection method. However, these devices do not enable high-frequency two- or three-dimensional measurements of a surface subjected to stress.

The present invention thus consists in a device which makes high-frequency measurements of a linear zone.

Thus, the present invention refers to a device able to make high-frequency measurements of the evolution of a same linear zone subjected to stress. More particularly, the present invention uses a laser-diode laser-line generator (10) which projects a laser line on the surface whose topography is to be determined. At the same time, the compressor delivers air to the line in order to deform the measurement zone.

The laser line is reflected on the CMos or CCD sensor (11) which divides it into a number of points proportional to the lateral resolution (laser line width/lateral resolution). By triangulation, the profilometer measures, in an absolute manner, the height of each point on the line in order to deduce, via the data-processing software, the mechanical, surface and volume properties of the measured zone.

The profilometer has a lateral resolution of less than 2 mm and axial resolution of less than 5 microns, and preferably around 1 micron or less. The measurement frequency is preferably greater than 50 Hz, for example 100 Hz, and may reach 1000 Hz. Thus, the position of the points on the laser line projected on the skin surface to be measured is recorded at a frequency of between 50 and 1000 Hz, and preferably about 250 Hz.

Therefore the interest of the laser line resides in the ability to obtain multipoint information (linear zone) over several centimeters within a single measurement and thus to avoid the artifacts that may be associated with a prompt measurement .

In order to extract the mechanical, surface and volume properties of the material, a number of adjustments and calculations using mathematical algorithms are necessary. Scientific calculation software enables processing of such data.

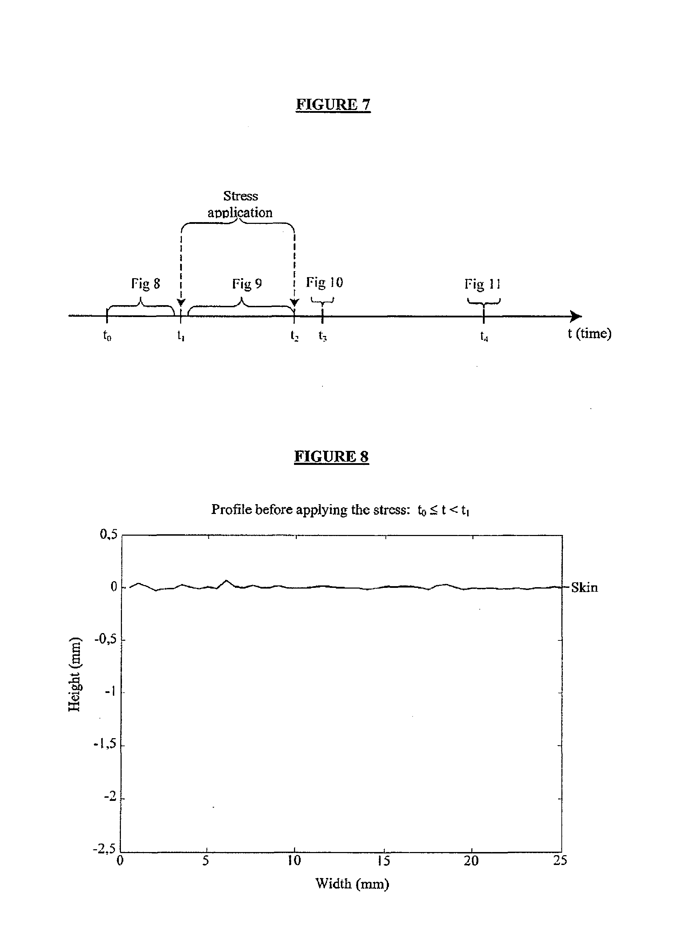

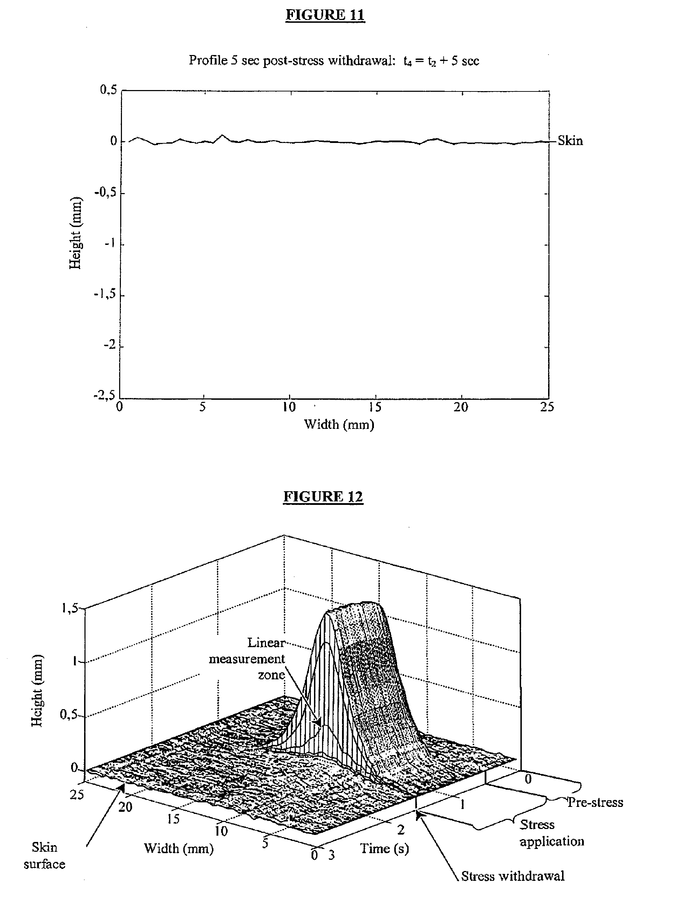

The measurements are thus transferred to EXCEL or MATLAB for processing. The EXCEL or MATLAB data are the measurements for each point of the linear zone for a given time (figures 8, 9, 10 and 11) and for all times (figure 12). The number of profiles per second depends on the acquisition frequency. At first, the data are rectified for exploitation. Since the system is very precise (linearity of plus or minus 15 µm), the slightest movement of the subject falsifies the results.

These points subsequently enable three-dimensional graphic exploitation and determination of the magnitude of the skin displacements.

In a single measurement, it is thus possible to obtain a linear zone profile, i.e. a two-dimensional spatial measurement of the linear zone at a given time (figures 8, 9, 10 and 11). The measurement is the absolute measurement of the distance between the laser diode and surface observed.

To facilitate comprehension of the invention, figures 7, 8, 9, 10, 11 and 12 are given as examples and explained below.

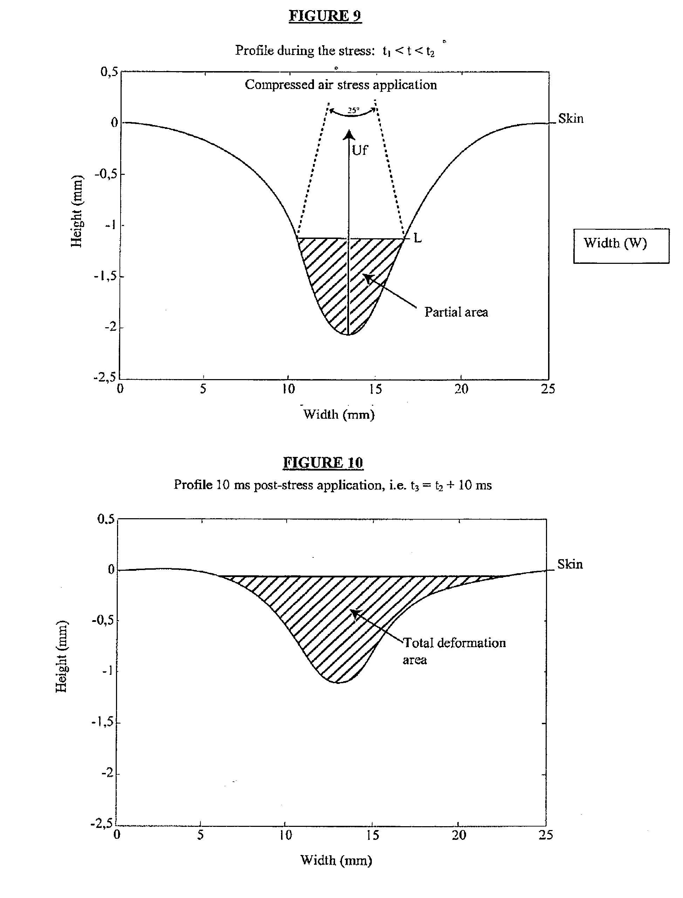

Figure 7 shows application of the compressed air stress on the skin as a function of time during a procedure at a frequency of 100 Hertz (Hz). Figures 8, 9, 10 and 11 are dependent on figure 7 and show the profiles measured at the various times. More particularly, figure 8 shows an example of the profile of a linear zone undergoing measurement before applying a stress, i.e. at a time t such that t₀ ≤ t < t₁. Figure 9 shows another example of the profile of the same linear zone during stress application, i.e. at a time t such that t₁ < t < t₂. Figure 10 also shows an example of the profile of the linear zone at t₃ = t₂ + 10 ms at a frequency of 100 Hz. Figure 11 shows an example of the profile of said linear zone 5 s after stress applying at t₄ = t₂ + 5 s.

In addition, the various profiles obtained during the procedure described in figure 7 showing the same linear zone subjected to a constant stress with measurement every 10 ms (pre-, per- and post-stress) are shown in figure 12. Figure 12 thus shows a breakdown of the cutaneous displacement of the same linear zone pre-, per- and post-application of the stress and every 10 ms.

Thus, one of the advantages of the present invention is the ability to measure the displacement profile of a linear zone subjected to compressed air stress with high-frequency measurement between 50 and 1000 Hz and preferably at about 250 Hz.

In another aspect of the invention, the device has the advantage of high-frequency measurement of various parameters directly related to the process of skin aging. Those parameters were difficult to obtain at high frequency before implementation of the present invention. The present invention enables measurement, at a frequency of between 50 and 1000 Hz, and preferably about 250 Hz, of the surface properties, tissue cohesion and volume properties of a measurement zone subjected to a stress at a given time.

### Measurement of surface properties and tissue cohesion (figures 9 ,10)

According to the present invention, it is possible to calculate a certain number of characteristics directly related to skin aging.

In fact, the scientific calculation software enables calculation of the area of displacement of each profile measured at high frequency (figure 9). It is thus possible to study, at high frequency, the change over time of the areas of the same zone measured pre-, per- and post-application of the stress.

It is also possible to investigate a fraction (L) of the maximum height (Uf) of a profile at a given time (figure 8).

With the present invention it is possible to obtain and thus to study a new parameter, which is the time course of tissue cohesion. Tissue cohesion directly reflects the ability of the material under study to absorb impacts. More particularly, when a force is exerted at constant pressure on young skin and on older skin, the young skin distributes the stress more widely than the older skin. The mechanical qualities of young skin enable diffusion of the incident pressure over a greater area than will do an older skin. By analogy, the mechanical properties of young skin may be compared to the mechanical properties of a trampoline while the mechanical properties of older skin may be compared to the mechanical properties of modeling clay. When a force is exerted on mature skin, the cutaneous tissue assumes a shape induced by the stress, in particular due to a decrease in the density of the extracellular matrix. The older the skin is the longer it takes to return to its initial shape. In contrast, young skin diffuses the stress experienced over all its surface and thus returns to its initial shape almost immediately: the means of decreasing the pressure of an impact consists in increasing the area subjected to the impact.

Tissue cohesion is a function of two parameters: Uf, the maximum amplitude of displacement, and W, the width of the deformation at a predefined fraction of the height.

Thus, the denser and more cohesive a tissue is, the better it absorbs compressive impacts (axial compression of the tissue). The greater the width is, the greater the distribution of the deformation, the greater the tissue cohesion and the more elastic the skin will be, and, hence, the younger the skin will be.

The present invention thus enables study of the total or partial area of skin displacement for each profile (figures 9 and 10) and hence the evolution, at high frequency, of these areas. In addition, the present invention enables obtaining a new parameter for measurement of the properties of the skin: the evolution at high frequency of tissue cohesion.

### Volume properties:

The use of the stress device without contact on the measurement zone enables imaging,
quantification and calculation of the amplitude of displacement in the three axes of an orthonormal system (x, y, z) and as a function of time.

The present invention is based on the hypothesis that the deformation is isotropic (since the stress is symmetrical). It is thus possible to extrapolate the volumes by 180-degree rotation of the profiles. A hollow derived from displacement of the measurement zone is thus obtained and represents the real calculated image of the volume of skin displacement.

As was the case for areas, the present invention enables obtaining the total volume or a fraction of that volume together with the volume of a profile at a given time or the evolution of the volume of skin displacement and at high frequency. Thus another advantage of the present invention is to make it possible to monitor the evolution of skin displacement volume, at high frequency.

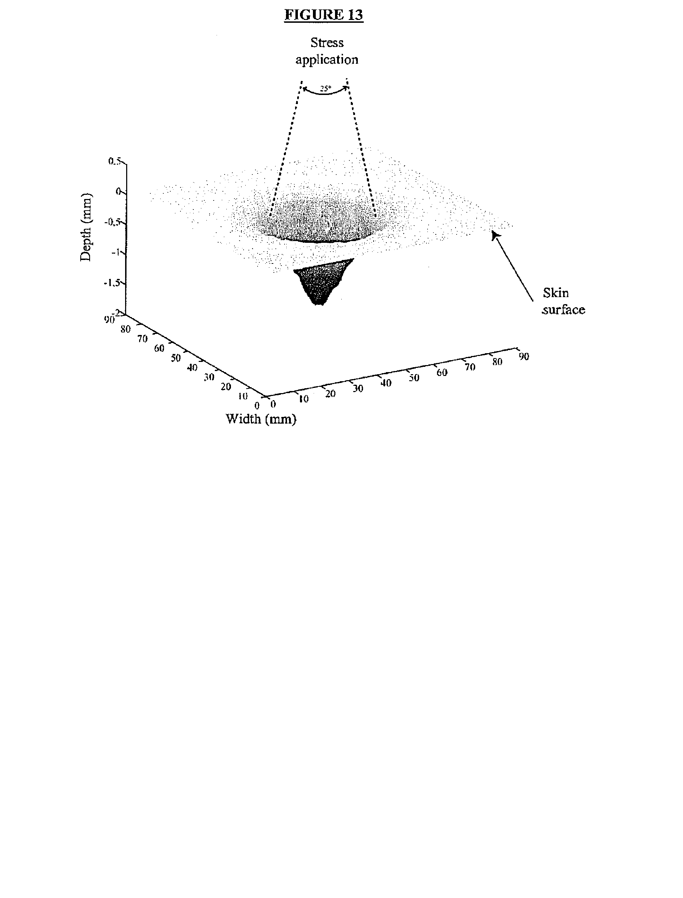

Figure 13 shows the volume of skin displacement.

### Measurement of mechanical properties:

In the context of the present invention, 'mechanical properties' means the physical parameters that can be defined from the displacement of a material subjected to a mechanical stress. All deformable materials, including the skin, have elastic, viscoelastic and plastic properties, i.e. a succession of responses to the applied stress.

Thus, the term 'elasticity' indicates the non-malleable part of the deformation under stress. The term 'plasticity' refers to the malleable part of the deformation under stress.

Burger's model enables the mechanical behavior of a material to be modeled.

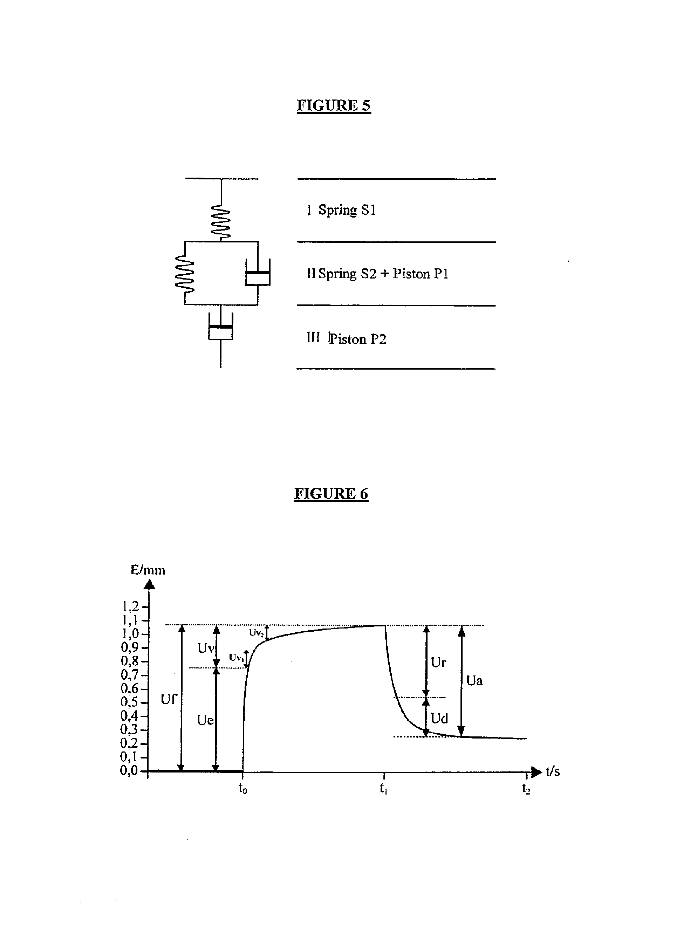

Figure 5 shows a scheme of this model symbolized by an assembly of springs and pistons. Figure 6 shows the evolution of displacement of a system (such as skin) whose behavior can be modeled using Burger's model in which:
Ue indicates elasticity,
Uv indicates viscoelasticity,
Uf indicates final deformation,
Ur designates immediate recovery,
Ud designates delayed recovery,
Ua designates added recovery.

Under strong and prolonged but reversible stress, the skin undergoes two successive types of elongation:
- the Ue phase of immediate extension, considered purely elastic, due to the elastic network of the dermis, to collagen bundles and to the deformability of the epidermis and of the stratum corneum,
- the viscosity (Uv) which includes Uv1 (a variable extension (creep)) and/or Uv2 (constant creep) which are due to the basic substance, the difficulty of liquids circulating between dermal fibers and friction between protein structures.

The limits between Ue and Uv1, and Uv1 and Uv2 are imprecise since they depend on the proportions of elastic and viscous resistance in the tissue, which vary depending on the site, age and subject.

When the stress is stopped, the skin imnediately retracts (Ur, purely elastic recovery), but in an incomplete manner in accordance with a logic similar to that of extension. More time is required for complete recovery (viscoelastic recovery phase). Recovery is fast in children and slower in the elderly.

In contrast, in the event of important extensions or compressions (injury), the deformation is plastic and irreversible (Agache et al., 2000).

Thus, according to the present invention high-frequency measurement of the elastic, plastic and viscoelastic properties of a linear zone of the skin subjected to stress is possible. In consequence, one of the advantages of the present invention is to enable fine measurement free from the artifacts that may derive from one-dimensional measurements and free from the confounding parameters that would result from direct contact between the system and measurement zone.

Thus, the present device is able to implement high-frequency measurement of a linear zone subjected to a stress while remaining free from all the parameters interfering with measurement. More particularly, the invention enables monitoring and recording using precise, continuous and reproducible measurements of the mechanical, surface and volume properties of skin displacement.

It is thus possible to continuously measure skin displacement and recovery to the equilibrium state in the fields of cosmetics, dermatology, human and animal diseases and/or nutraceuticals. The device enables high-frequency measurement of the evolution of the area, volume and tissue cohesion of a zone subjected to a stress. Thus, the system enables, from a linear zone subjected to a stress, deduction of the surface properties, tissue cohesion and volume properties of the zone under stress.

The device also enables study of the mechanical properties and particularly the elastic, plastic, viscoelastic, tone, fatigue, recovery and suppleness properties of a linear zone of the skin at high frequency. In the context of the present invention, the term 'tone' indicates the ability of the skin to rapidly return to an equilibrium state when the stress is withdrawn.

Thus, the present invention enables monitoring of the natural or induced aging of said material. The physiological or pathological changes in the skin related, for example, to natural skin aging (chronological) or induced skin aging (medicinal treatment, ultraviolet radiation, environment, pollution, hygiene products, medical devices, cicatrization, infrared radiation, cold, dry air, foods, toxic substances) or cosmetic treatments can thus be followed up and evaluated. The process is non-invasive and non-destructive.

The device enables monitoring pathological skin in man or animals and monitoring of the course or regression of disease by monitoring the changes in mechanical parameters over time or following topical or oral treatment. The device may be used to diagnose diseases directly or indirectly affecting the mechanical properties of the skin, and, if necessary, to enable orientation toward the treatments most appropriate for the condition of the skin or its characteristics.

The device also enables evaluation, on healthy or pathological skin, of the direct or indirect effect of medical devices or massages on the mechanical properties of the skin associated or not associated with application or intake of a product or use of luminotherapy.

The device also enables evaluation of the direct or indirect modifications of healthy or pathological skin induced by medicinal products, dermatological products, cosmetic products, nutraceuticals, cosmetics (moisturizing products, sunscreens, anti-wrinkle products, firming products) and foods.

According to another aspect, the present invention enables measurement of the evolution of the mechanical, surface and volume properties of all deformable surfaces or materials such as the skin, deformable polymers, foams and elastomers.

The device will also be of value in specialized industries such as the following:
- polymer industry: solids, gels and foams;
- construction industry: building materials (insulators, anti-vibration products);
- medical device industry (e.g. rubber prostheses);
- food industry (viscosity of food products);
- chemical industry (fertilizers, pesticides);
- elastomer industry.

## Claims

**1.** Device for measuring the mechanical properties of the skin comprising:
a) A pneumatic assembly (16) configured to deliver compressed air to apply a stress to the surface of the skin undergoing measurement,
b) An electronic assembly (6) configured for contact-free measurements on said surface of the skin subjected to the stress, and
c) A control software (19) configured to control the electronic assembly (6) and to process measurement data in order to deduce the mechanical properties of the skin,
**characterized in that** the electronic assembly comprises a laser-line profilometer (6) comprising:
- a laser-diode laser-line generator (10) configured to project a laser line on said skin surface, and
- a CMos or CCD sensor (11) configured to record the evolution of the position of said laser line points, said profilometer (6) being adapted to measure the position of the laser line at frequencies of between 50 and 1000 Hz.

**2.** Device according to claim 1, **characterized in that** the frequency of measurement is 100 Hz.

**3.** Device according to claim 1 or 2, **characterized in that** the linearity of the profilometer (6) is less than 15 µm, and preferably 1 µm, and the axial resolution of the profilometer (6) is less than 5 µm, and preferably 1 µm.

**4.** Device according to anyone of claims 1 to 3, **characterized in that** the lateral resolution of the profilometer (6) is less than 2 mm.

**5.** Device according to claim 4, **characterized in that** the lateral resolution is between 10 and 100 µm.

**6.** Device according to anyone of claims 1 to 5, **characterized in that** the pneumatic assembly (16) configured to deliver compressed air to apply a stress to the surface of the skin comprises an air compressor (1) supplying a nozzle (9) via a pneumatic switch (2), a flow regulator (4) and a pressure regulator (3)

**7.** Device according to claim 6, **characterized in that** said nozzle (9) is orientable by means of an optomechanical tilt stage (7).

**8.** Non-invasive and non-destructive method for measuring the mechanical surface and volume properties of the skin, comprising :
a) Applying a deforming stress to the skin surface under measurement by using an air jet with adjustable pressure and flow rate;
**characterized in that** it comprises the following steps:
b) Projecting a laser line on the surface of the skin deformed by said stress,
c) Recording the position of the laser line points at a frequency of between 50 and 1000 Hz without contact with said skin surface,
d) Processing the data recorded in step c) using a software to deduce the mechanical, surface and volume properties of the skin.

**9.** Method according to claim 8, **characterized in that** the frequency of recording of the position of the laser line points or profile is at a frequency of 100 Hz.

**10.** Method according to claim 8 or 9, **characterized in that** the linearity of the measurement is less than 15 µm, and preferably 1 µm, and the axial resolution of the measurement is less than 5 µm, and preferably 1 µm.

**11.** Method according to anyone of claims 8 to 10, **characterized in that** the lateral resolution of the measurement is less than 2 mm.

**12.** Method according to claim 11, **characterized in that** the lateral resolution is between 10 and 100 µm.

**13.** Use of the device according to anyone of claims 1 to 7 for deducing the surface properties, tissue cohesion and volume properties of the zone subjected to a stress.

**14.** Use of the device according to anyone of claims 1 to 7 for high-frequency measuring of the mechanical properties and, particularly, the elastic, plastic and viscoelastic properties of a linear zone subjected to a stress.

**15.** Use of the device according to anyone of claims 1 to 7 and/or the method according to anyone of claims 8 to 12 in the fields of cosmetics, dermatology, human health, animal health and/or nutraceuticals.

**16.** Use of the method according to anyone of claims 8 to 12 for monitoring the natural or induced aging of materials, or the physiological or pathological modifications of the skin related, for example, to natural or induced skin aging, and for cosmetic treatments.

## Patentansprüche

1. Vorrichtung zum Messen der mechanischen Eigenschaften der Haut umfassend:
a) eine Druckluftanordnung (16), welche dazu geeignet und ausgebildet ist, Druckluft zu übertragen, um eine zu untersuchte Hautoberfläche zu belasten,
b) eine Elektronikanordnung (6), welche dazu geeignet und ausgebildet ist, berührungslose Messungen an der belasteten Hautoberfläche durchzuführen,
c) eine Kontrollsoftware (19), welche dazu geeignet und ausgebildet ist, die Elektronikanordnung (6) zu kontrollieren und die Messdaten zu verarbeiten, um Rückschlüsse auf die mechanischen Eigenschaften der Haut zu ziehen,
**dadurch gekennzeichnet, dass** die Elektronikanordnung ein Laserlinien-Profilometer (6) umfasst, welches aufweist:
- einen Laserdioden-Laserlinienerzeuger (10), der dazu geeignet und ausgebildet ist, eine Laserlinie auf die Hautoberfläche zu projizieren, und
- einen CMos oder CCD Sensor (11), welcher dazu geeignet und ausgebildet ist, den Verlauf der Position der Punkte der Laserlinie aufzuzeichnen, wobei das Profilometer (6) dazu geeignet und ausgebildet ist, die Position der Laserlinie bei einer Frequenz zwischen 50 und 1000 Hz zu messen.

2. Vorrichtung nach Anspruch 1, **dadurch gekennzeichnet, dass** die Messefrequenz 100 Hz beträgt.

3. Vorrichtung nach Anspruch 1 oder 2, **dadurch gekennzeichnet, dass** die Linearität des Profilometers (6) kleiner als 15 µm und bevorzugt kleiner als 1 µm ist und dass die axiale Auflösung des Profilometers (6) kleiner als 4 µm und bevorzugt 1 µm ist.

4. Vorrichtung nach einem der Ansprüche 1 bis 3, **dadurch gekennzeichnet, dass** die laterale Auflösung des Profilometers (6) kleiner als 2 mm ist.

5. Vorrichtung nach Anspruch 4, **dadurch gekennzeichnet, dass** die laterale Auflösung zwischen 10 und 100 µm liegt.

6. Vorrichtung nach einem der Ansprüche 1 bis 5, **dadurch gekennzeichnet, dass** die Druckluftanordnung (16), welche dazu geeignet und ausgebildet ist, Druckluft auf die Hautoberfläche zu übertragen, um diese zu belasten, einen Luftkompressor (1) der eine Düse (9) mittels einer Druckluftweiche (2), einen Volumenstromregler (4) und einen Druckregler (3) umfasst.

7. Vorrichtung nach Anspruch 6, **dadurch gekennzeichnet, dass** die Düse (9) über einen optomechanischen Neigetisch ausrichtbar ist.

8. Nichtinvasives und nichtzerstörendes Verfahren zur Messung der mechanischen Oberfläche und der Volumeneigenschaften der Haut, umfassend:
a) die Erzeugung einer Verformungsbeanspruchung an einer zu untersuchenden Hautoberfläche mittels eines Druckluftstroms mit einstellbarem Druck und Volumenstrom, **dadurch gekennzeichnet dass** das Verfahren die folgenden Schritte umfasst:
b) projizieren einer Laserlinie auf die Oberfläche der Haut, die durch die Beanspruchung verformt ist,
c) aufzeichnen der Position der Punkte der Laserlinie bei einer Frequenz zwischen 50 und 1000 Hz,
d) verarbeiten der in Schritt c) aufgezeichneten Daten mittels einer Software, um Rückschlüsse auf die mechanischen Eigenschaften, die Oberflächeneigenschaften und die Volumeneigenschaften der Haut zu ziehen.

9. Verfahren nach Anspruch 8, **dadurch gekennzeichnet, dass** die Aufzeichnungsfrequenz der Position der Punkte der Laserlinie und des Profils der Laserlinie bei einer Frequenz von 100 Hz erfolgt.

10. Verfahren nach Anspruch 8 oder 9, **dadurch gekennzeichnet, dass** die Linearität der Messung kleiner als 15 µm und bevorzugt 1 µm ist und dass die axiale Auflösung der Messung kleiner als 5 µm und bevorzugt 1 µm ist.

11. Verfahren nach einem der Ansprüche 8 bis 10, **dadurch gekennzeichnet, dass** die laterale Auflösung der Messung kleiner als 2 mm ist.

12. Verfahren nach Anspruch 11, **dadurch gekennzeichnet, dass** die laterale Auflösung zwischen 10 und 100 µm ist.

13. Verwendung der Vorrichtung nach einem der Ansprüche 1 bis 7, um Rückschlüsse auf die Oberflächeneigenschaften, den Gewebezusammenhalt und die Volumeneigenschaften der einer Beanspruchung ausgesetzten Zone zu ziehen.

14. Verwendung der Vorrichtung nach einem der Ansprüche 1 bis 7 zur Hochfrequenzmessung von mechanischen Eigenschaften und insbesondere der Elastizität, der plastischen und der viskoelastischen Eigenschaften eines linearen Bereichs, der einer Beanspruchung ausgesetzt ist.

15. Verwendung der Vorrichtung nach einem der Ansprüche 1 bis 7, und/oder des Verfahrens nach einem der Ansprüche 8 bis 12 im Bereich von Kosmetika, Dermatologie, der Gesundheit von Menschen, der Gesundheit von Tieren und/oder Nahrungsmittelergänzungsprodukten.

16. Verwendung des Verfahrens nach einem der Ansprüche 9 bis 12, um das natürliche oder das induzierte Altern von Materialien zu überwachen oder um physiologische oder pathologische Veränderungen der Haut, beispielsweise betreffend die natürliche oder induzierte Hautalterung, zu überwachen und für kosmetische Behandlungen.

## Revendications

1. Dispositif de mesure des propriétés mécaniques de la peau, comprenant :
a) un ensemble pneumatique (16) configuré pour délivrer de l'air comprimé pour appliquer une contrainte sur une surface de peau soumise à des mesures,
b) un ensemble électronique (6) configuré pour procéder à des mesures sans contact sur ladite surface de peau soumise à la contrainte, et
c) un logiciel de pilotage (19) configuré pour contrôler l'ensemble électronique (6) et pour
traiter des données de mesure pour en déduire les propriétés mécaniques de la peau, **caractérisé en ce que** l'ensemble électronique comprend un profilomètre à ligne laser (6) comprenant :
- un générateur de ligne laser à diode laser (10) configuré pour projeter une ligne laser sur ladite surface de peau, et
- un capteur CMos ou CCD (11) configuré pour enregistrer l'évolution de la position des points de ladite ligne laser, ledit profilomètre étant adapté à mesurer la position de la ligne laser à des fréquences allant de 50 à 1000 Hz.

2. Dispositif selon la revendication 1, **caractérisé en ce que** la fréquence de mesure est de 100 Hz.

3. Dispositif selon la revendication 1 ou 2, **caractérisé en ce que** la linéarité du profilomètre (6) est inférieure à 15 µm, et de préférence de 1µm, et la résolution axiale du profilomètre (6) est inférieure à 5 µm, et de préférence de 1 µm.

4. Dispositif selon l'une quelconque des revendications 1 à 3, **caractérisé en ce que** la résolution latérale du profilomètre (6) est inférieure à 2 mm.

5. Dispositif selon la revendication 4, **caractérisé en ce que** la résolution latérale est comprise entre 10 et 100 µm.

6. Dispositif selon l'une quelconque des revendications 1 à 5, **caractérisé en ce que** l'ensemble pneumatique (16) configuré pour délivrer de l'air comprimé pour appliquer une contrainte sur la surface de la peau comprend un compresseur d'air (1) qui alimente une buse (9) par l'intermédiaire d'un interrupteur pneumatique (2), un régulateur de débit (4) et un régulateur de pression (3).

7. Dispositif selon la revendication 6, **caractérisé en ce que** ladite buse (9) est orientable au moyen d'une plateforme de positionnement opto-mécanique d'inclinaison (7).

8. Procédé non-invasif et non-destructif de mesure des propriétés mécaniques, surfaciques et volumiques de la peau, comprenant :
a) l'application d'une contrainte déformante sur une surface de la peau soumise à la mesure au
moyen d'un jet d'air à pression et débit réglables ;
**caractérisé en ce qu'**il comprend les étapes suivantes :
b) la projection d'une ligne laser sur la surface de la peau déformée par ladite contrainte,
c) l'enregistrement de la position des points de la ligne laser à une fréquence comprise entre 50 et 1000 Hz sans contact avec ladite surface de la peau,
d) traiter les données enregistrées à l'étape c) au moyen d'un logiciel pour en déduire les propriétés mécaniques, surfaciques et volumiques de la peau.

9. Procédé selon la revendication 8, **caractérisé en ce que** la fréquence d'enregistrement de la position des points de la ligne laser ou profil est de 100 Hz.

10. Procédé selon la revendication 8 ou 9, **caractérisé en ce que** la linéarité des mesures est inférieure à 15 µm, et de préférence de 1µm, et la résolution axiale de la mesure est inférieure à 5 µm, et de préférence de 1 µm.

11. Procédé selon l'une quelconque des revendications 8 à 10, **caractérisé en ce que** la résolution latérale de la mesure est inférieure à 2 mm.

12. Procédé selon la revendication 11, **caractérisé en ce que** la résolution latérale est comprise entre 10 et 100 µm.

13. Utilisation du dispositif selon l'une quelconque des revendications 1 à 7 pour déduire les propriétés de surface, la cohésion du tissu et les propriétés volumiques de la zone soumise à une contrainte.

14. Utilisation du dispositif selon l'une quelconque des revendications 1 à 7 pour mesurer à fréquences élevées les propriétés mécaniques et, en particulier, les propriétés élastiques, plastiques et visco-élastiques, d'une zone linéaire soumise à une contrainte.

15. Utilisation d'un dispositif selon l'une quelconque des revendications 1 à 7 et/ou du procédé selon l'une quelconque des revendications 8 à 12, dans les domaines des cosmétiques, de la dermatologie, de la pathologie animale ou humaine et/ou de la nutraceutique.

16. Utilisation du procédé selon l'une quelconque des revendications 8 à 12 pour suivre le vieillissement naturel ou induit de matériaux, ou les modifications physiologiques ou pathologiques de la peau liées, par exemple, au vieillissement cutané naturel ou induit, ainsi que les traitements cosmétiques.
